# EUROPEAN PATENT APPLICATION

(11) **EP 4 686 470 A1**
(43) Date of publication of application: **04.02.2026**
(21) Application number: 24192298.8
(22) Date of filing: 01.08.2024
(51) Int. Cl.: A61K 31/12, C07K 5/00

(54) **MACROCYCLIC PEPTIDOMIMETICS AS ANTIVIRAL AGENTS**

(71) Applicant: Ospedale San Raffaele S.r.l., 20132 Milano (IT)
(72) Inventor: SUMMA, Vincenzo, 00071 Viale Priamo 44 - Pomezia (RM) (IT); CANNALIRE, Rolando, 72021 Via Sant Eligio 7, Brindisi (IT); DE FRANCESCO, Raffaele, 20146 Via Leone Tolstoi 1, Milano (MI) (IT); DONNICI, Lorena, 20151 Via Pier Paolo Pasolini 19 , Milano (MI) (IT); IANNACONE, Matteo, 20129 Via Verrocchio 2 - Milano (MI) (IT); GUIDOTTI, Luca, 20131 Via Gozzano 4 - Milano (MI) (IT)
(74) Representative: Pace Napoleone, Maria

(57) **Abstract**

The present invention refers to macrocyclic peptides as covalent reversible inhibitors of SARS-CoV-2 M^{pro} with a potential broad-spectrum activity against CoV proteases. The compounds are therefore indicated for treating M^{pro} associated or mediated diseases and conditions, such as severe acute respiratory syndrome coronavirus 2 (SARS-CoV2).

## Description

### FIELD OF THE INVENTION

The present invention relates to novel macrocyclic peptides as covalent reversible inhibitors of SARS-CoV-2 M^{pro} with a potential broad-spectrum activity against CoV proteases. The compounds of the invention significantly differ from known inhibitors particularly for the P1 residue, establishing a new pattern of interactions in the S 1 subpocket of the enzyme. The compounds are therefore indicated for use in the treatment and/or prevention of a viral infection, preferably for treating M^{pro} associated or mediated diseases and conditions, such as severe acute respiratory syndrome coronavirus 2 (SARS-CoV2).

### BACKGROUND

SARS-COV-2 (Severe Acute Respiratory Syndrome Coronavirus 2) is the causative agent of COVID-19 pandemic, with an estimated over 774 million confirmed cases and more than seven million deaths reported globally, according by WHO as of 4 February 2024. (https://www.who.int/publications/m/item/covid-19-epidemiological-update-16-february-2024). Despite the introduction of vaccines and drugs, the circulation of the virus persists, and the need for safe and effective antiviral agents is emphasized, especially with the emergence of new SARS-CoV-2 variants. The genome of SARS-CoV-2 revealed several potential targets for the inhibition of viral replication. To date, only three Direct Acting Antivirals (DAAs) have been discovered and approved worldwide, to fight the COVID-19 pandemic. Specifically, Remdesivir (dosed i.v.) and Molnupiravir (dosed p.o.) are repurposed prodrug nucleotide inhibitors of the viral RNA-dependent RNA polymerase (RdRp) with good efficacy; Nirmatrelvir (dosed p.o.) represents a step forward, being the first-in-class compound approved as reversible covalent inhibitor of SARS-CoV-2 Main protease (M^{pro}), for administration in combination with Ritonavir, a "PK booster" (trade name Paxlovid) ^{2,3}. The structures of the compounds are reported in the scheme 1 below:

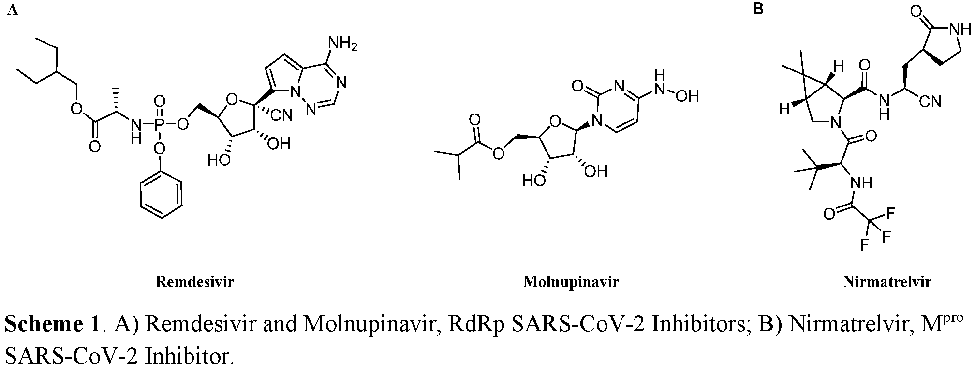

As can be seen Nirmatrelvir, the first-in-class M^{pro} inhibitor, is characterized by a γ-lactamic ring in P1, which is able to fit into the S1 pocket and interacts with His₁₆₃ and Glu₁₆₆, a dimethylbicycloproline group in P2, resulting in extensive van der Waals interactions in the hydrophobic S2 pocket. The capping trifluoromethyl group, instead, is important to anchor the inhibitor at the S4 subpocket by forming a stabilizing contact with Gln₁₉₂. The nitrile warhead is able to form a reversible covalent thioimidate adduct with the sulfur atom of the Cys₁₄₅ at S1'.

The M^{Pro} attracted significant interest from the earliest stages of the pandemic to design specific and safe new inhibitors. First, because of its essential biological role in the viral replication, cleaving the SARS-CoV-2 polyprotein after translation to release functional viral proteins; secondly, it has no human homologues and the active site of this cysteine protease is highly conserved across the *orthocoronaviridae* subfamily, allowing the identification and development of Pan CoVs inhibitors. Taking advantage of a non-canonical active site catalytic dyad, Cys₁₄₅-His₄₁, and the substrate sequence at P2-P1-P1', Leu-Gln-Ser (Ala, Gly), the design of the new inhibitors has been focused on the synthesis of compounds with a peptidomimetic structure, mimicking the natural substrate, endowing an electrophilic warhead able to react with the nucleophilic catalytic Cys₁₄₅, blocking the enzyme almost irreversibly, thanks to a kinetic characterized by a fast kₒₙ and a very slow K_{off}, acting as covalent reversible inhibitors².

Due to the intrinsic value to target the M^{Pro}, the global effort in identifying new candidates produced four molecules in clinical development (phase 2/3 active): *Pomotrelvir*⁴ (from Pardes Biosciences, M^{pro} IC₅₀ = 14 nM), *Bofutrelvir*⁵ (from Frontier Biotechnologies, M^{pro} IC₅₀ = 53 nM) and *Ibuzatrelvir*⁶ (from Pfizer, administered without Ritonavir) shared a linear peptidomimetic structure with nitrile or aldehyde warhead, *EDP-235*⁷ (from Enanta Pharmaceuticals, M^{pro} IC₅₀ = 5.8 nM) has a macrocyclic peptidomimetic structure, exploiting the condensation between P2-P1 residues, aiming to stabilize the ligand's bioactive conformation. The compound *EDP-235* reduced some symptoms of the respiratory disease but did not reduce viral load or time to improvement when compared to placebo (*https:*//*ir.enanta.com*/*news-releases*/*news-release-details*/*enanta-pharmaceuticals-reports-positive-topline-results-phase-2*). *Ensitrelvir*⁸ (from Shionogi, M^{pro} IC₅₀ = 13 nM), approved only in Japan, differs from the others because is the only non-peptidomimetic compound in clinical trials. The structures of the above compounds are reported in Scheme 2 below:

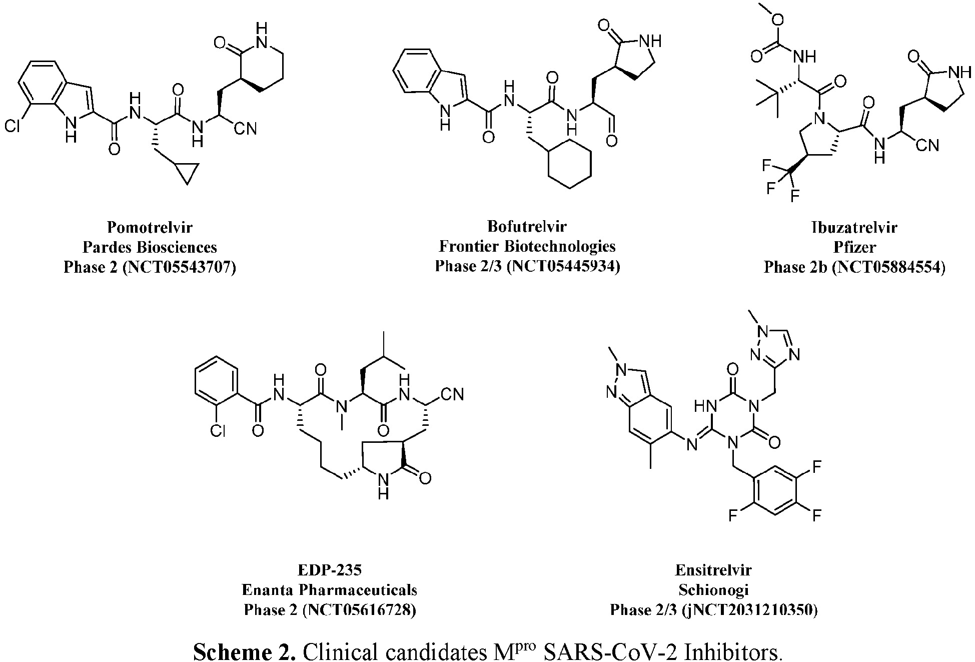

Therefore an ongoing need exists for the development of novel M^{pro} inhibitors with cellular efficacy, metabolic stability and good pharmacokinetic properties. The present invention provides compounds significantly differing from the M^{pro} inhibitors reported so far, particularly for the P1 residue. Indeed, the compounds of the invention possess an unusual D-proline, bound to the side-chain carboxylate of a Glu residue, to mimic the Gln in P1, instead of the classic γ-lactam. Consequently, they have been predicted to establish a new pattern of interactions in the S 1 subpocket of the enzyme.

### SUMMARY OF THE INVENTION

The present invention provides novel M^{pro} inhibitor, rationally designed from the understanding of the key interactions involved in the binding of substrates in the active site of M^{pro}. Compounds of the invention are macrocyclic inhibitors with ring size from 15 to 18 terms and designed to interact in the binding pocket of the SARS-COV-2 M^{pro}. The compounds share a linear tripeptide backbone joined through the side chains of the first and the third aminoacid via a proline residue or a derivative thereof. The present compounds are characterized by the proline residue in P1, able to engage a hydrogen bond with the carbonyl group of His₁₆₃, a Leu in P2 as in the natural substrate and a nitrile warhead as in Nirmaltrevir. The compounds optionally possess a further functionalization as P3 and can be methylated on the leucine nitrogen thus having an additional constrain in the macrocyclic structure.

It is therefore an object of the invention a compound of general formula (I): 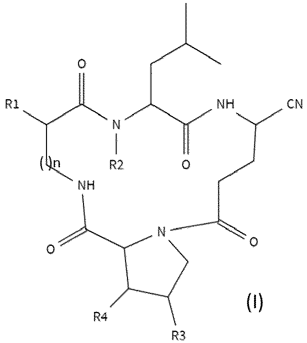 wherein:
n is comprised between 1 and 4;
R₁ is H or R₅C(=O)NH;
R₂ is H or CH₃
R₃ and R₄ are each independently selected from H, halogen, C₁₋₃alkyl, OH, C₁₋₃haloalkyl, or R₃ and R₄ are linked together to form a fused C₃₋₆-cyclic ring or a C₃₋₆-heterocyclic ring each of said ring being optionally substituted with one or more substituents selected from methyl, halogen;
R₅ is selected from C₁₋₄alkyl, C₁₋₄alkyl-aryl, C₁₋₄alkyl-heteroaryl, aryl or heteroaryl wherein each of said aryl and heteroaryl is optionally substituted with one or more substituents selected from halogen, C₁₋₃alkyl, OH, C₁₋₃haloalkyl;
and stereoisomers and pharmaceutically acceptable salt thereof.

In a preferred embodiment the compound of formula (I) has general formula (IA): 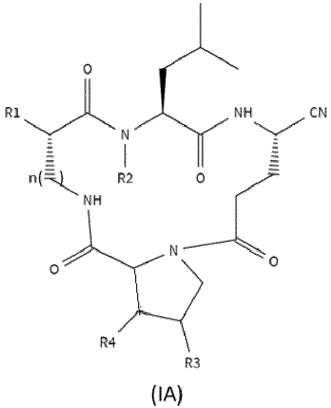 and pharmaceutically acceptable salts thereof.

In a further preferred embodiment the compound has general formula (IB): 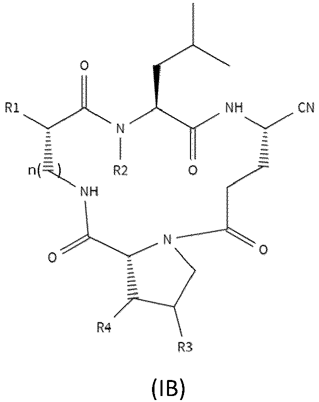 and pharmaceutically acceptable salts thereof.

Preferably in the compound of formula (I), (IA) and (IB) R₁ is R₅C(=O)NH and/or R₅ is a pyridine, more preferably R₅ is 2-pyridine.

Preferably in the compound of formula (I), (IA) and (IB) R₃ and R₄ are H.

Preferably the compound of formula (I), (IA) and (IB) is selected from:
- (7S,10S,17aR)-7-isobutyl-1,5,8,13-tetraoxohexadecahydro-1H-pyrrolo[1,2-a][1,4,8,11]tetraazacyclopentadecine-10-carbonitrile
- (8S,11S,18aR)-8-isobutyl-1,6,9,14-tetraoxooctadecahydropyrrolo[1,2-a][1,4,9,12]tetraazacyclohexadecine-11-carbonitrile
- (9S,12S,19aR)-9-isobutyl-1,7,10,15-tetraoxooctadecahydro-1H-pyrrolo[2,1-c][1,4,9,12]tetraazacycloheptadecine-12-carbonitrile
- N-((4S,7S,10S,17aR)-10-cyano-7-isobutyl-1,5,8,13-tetraoxohexadecahydro-1H-pyrrolo[1,2-a][1,4,8,11]tetraazacyclopentadecin-4-yl)picolinamide
- N-((5S,8S,11S,18aR)-11-cyano-8-isobutyl-1,6,9,14-tetraoxooctadecahydropyrrolo[1,2-a][1,4,9,12]tetraazacyclohexadecin-5-yl)picolinamide
- N-((6S,9S,12S,19aR)-12-cyano-9-isobutyl-1,7,10,15-tetraoxooctadecahydro-1H-pyrrolo[2,1-c][1,4,9,12]tetraazacycloheptadecin-6-yl)picolinamide
- N-((7S,10S,13 S,20aR)-13-cyano-10-isobutyl-1,8,11,16-tetraoxoicosahydropyrrolo[2,1-c][1,4,9,12]tetraazacyclooctadecin-7-yl)picolinamide
- N-((4S,7S,10S,17aR)-10-cyano-7-isobutyl-6-methyl-1,5,8,13-tetraoxohexadecahydro-1H-pyrrolo[1,2-a][1,4,8,11]tetraazacyclopentadecin-4-yl)picolinamide
- N-((5S,8S,11S,18aR)-11-cyano-8-isobutyl-7-methyl-1,6,9,14-tetraoxooctadecahydropyrrolo[1,2-a][1,4,9,12]tetraazacyclohexadecin-5-yl)picolinamide
- N-((6S,9S,12S,19aR)-12-cyano-9-isobutyl-8-methyl-1,7,10,15-tetraoxooctadecahydro-1H-pyrrolo[2,1-c][1,4,9,12]tetraazacycloheptadecin-6-yl)picolinamide
- N-((7S,10S,13S,20aR)-13-cyano-10-isobutyl-9-methyl-1,8,11,16-tetraoxoicosahydropyrrolo[2,1-c][1,4,9,12]tetraazacyclooctadecin-7-yl)picolinamide
and pharmaceutically acceptable salts thereof.

According to an embodiment of the invention a compound of formula (I), (IA) and (IB) is an inhibitor of the Main protease of SARS-COV2.

It is a further object of the invention a compound of formula (I), (IA) and (IB) for medical use, preferably for use in the treatment and/or prevention of a viral infection, preferably wherein said viral infection is an infection by an RNA virus, preferably the RNA virus is a Coronavirus, Picornavirus, Hepevirus, Influenza virus or Rhinovirus, preferably the Coronavirus is selected from the group consisting of MERSr-CoV, SARS-CoV-1, SARS-CoV-2, HCoV-OC43, HCoV-229E, HCoV- NL63, and HCoV-HKU1, preferably the Coronavirus is SARS-CoV-1 and/or SARS-CoV-2

It is a further object of the invention a pharmaceutical composition comprising the compound of formula (I), (IA) and (IB) as above defined and at least one excipient and optionally a further therapeutic agent.

Preferably said pharmaceutical composition is for use in the treatment and/or prevention of a viral infection, wherein said viral infection is an infection by an RNA virus, preferably the RNA virus is a Coronavirus, Picornavirus, Hepevirus, Influenza virus, Rhinovirus, preferably said Coronavirus is selected from the group consisting of MERSr-CoV, SARS-CoV-1, SARS-CoV-2, HCoV-OC43, HCoV-229E, HCoV- NL63, and HCoV-HKU1, preferably the Coronavirus is SARS-CoV-1 and/or SARS-CoV-2

It is a further object of the invention a method for inhibiting in vitro the M^{pro}, comprising contacting the cell with a compound of formula (I), (IA) and (IB) as above defined.

### DESCRIPTION OF THE INVENTION

The aim of the project was the design and synthesis of a new series of macrocycles peptides as covalent reversible inhibitors of SARS-CoV-2 M^{pro} with a broad-spectrum activity against coronaviruses proteases.

Two viral proteases are essential and responsible for processing the polyproteins: the main protease (M^{pro} or 3CL protease; ACS Pharmacol Transl Sci .2021 Mar 11;4(3):1096-1110. doi: 10.1021/acsptsci.0c00216. eCollection 2021 Jun 11; NCBI Reference Sequence: YP_009725301.1) and a papain-like protease (Hilgenfeld R. From SARS to MERS: crystallographic studies on coronaviral proteases enable antiviral drug design. FEBS J.2014;281(18):4085-96). Importantly, M^{pro} cleaves polypeptides after a glutamine residue in the P1 position of the substrate, which is a unique activity not observed in other human proteases and suggests that this viral protease can be specifically and selectively inhibited by a small molecule inhibitor (Zhang L et al. α-Ketoamides as Broad-Spectrum Inhibitors of Coronavirus and Enterovirus Replication: Structure-Based Design, Synthesis, and Activity Assessment. J Med Chem.2020;63(9):4562-4578).

Compounds of the present invention are potent inhibitors of M^{pro}, exhibit significant metabolic stability, and are useful in oral dosing to patients for treatment of COVID-19 and for prophylaxis against COVID-19.

Formula (I) compounds of the present invention show unexpected and dramatic improvements in antiviral activities as illustrated by the examples herein.

### Definitions

"Alkyl" refers to straight or branched chain hydrocarbyl including from 1 to about 20 carbon atoms. For instance, an alkyl can have from 1 to 10 carbon atoms or 1 to 6 carbon atoms. Exemplary alkyl includes straight chain alkyl groups such as methyl, ethyl, propyl, butyl, pentyl, hexyl, heptyl, octyl, nonyl, decyl, undecyl, dodecyl, and the like, and also includes branched chain isomers of straight chain alkyl groups, for example without limitation, -CH(CH₃)₂, -CH(CH₃)(CH₂CH₃), -CH(CH₂CH₃)₂, -C(CH₃)₃, -C(CH₂CH3)₃, -CH₂CH(CH₃)₂, -CH₂CH(CH₃)(CH₂CH₃), -CH₂CH(CH₂CH₃)₂, - CH₂C(CH₃)₃, -CH₂C(CH₂CH₃)₃, -CH(CH₃)CH(CH₃)(CH₂CH₃), -CH₂CH₂CH(CH₃)₂, - CH₂CH₂CH(CH₃)(CH₂CH₃), -CH₂CH₂CH(CH₂CH₃)₂, -CH₂CH₂C(CH₃)₃, -CH₂CH₂C(CH₂CH₃)₃, - CH(CH₃)CH₂CH(CH₃)₂, -CH(CH₃)CH(CH₃)CH(CH₃)₂, and the like. Thus, alkyl groups include primary alkyl groups, secondary alkyl groups, and tertiary alkyl groups. Preferred alkyl group of the invention include 1 to 3 carbon atoms. A preferred alkyl is methyl. An alkyl group can be unsubstituted or optionally substituted with one or more substituents as described herein.

Each of the terms "halogen," "halide," and "halo" refers to -F or fluoro, -Cl or chloro, -Br or bromo, or -I or iodo.

The term "alkenyl" refers to straight or branched chain hydrocarbyl groups including from 2 to about 20 carbon atoms having 1-3, 1-2, or at least one carbon to carbon double bond. An alkenyl group can be unsubstituted or optionally substituted with one or more substituents as described herein.

"Alkyne or "alkynyl" refers to a straight or branched chain unsaturated hydrocarbon having the indicated number of carbon atoms and at least one triple bond. Examples of a (C₂-C₈)alkynyl group include, but are not limited to, acetylene, propyne, 1-butyne, 2-butyne, 1- pentyne, 2-pentyne, 1-hexyne, 2-hexyne, 3-hexyne, 1-heptyne, 2-heptyne, 3-heptyne, 1-octyne, 2-octyne, 3-octyne and 4-octyne. An alkynyl group can be unsubstituted or optionally substituted with one or more substituents as described herein.

The term "alkoxy" or "alkoxyl" refers to an -O-alkyl group having the indicated number of carbon atoms. For example, a (C₁-C₆)-alkoxy group includes -O-methyl, -O-ethyl, -O-propyl, -O-isopropyl, -O-butyl, -O-sec-butyl, -O-tert-butyl, -O-pentyl, -O-isopentyl, -O-neopentyl, -O- hexyl, -O-isohexyl, and -O-neohexyl.

The term "cycloalkyl" refers to a saturated monocyclic, bicyclic, tricyclic, or polycyclic, 3- to 14-membered ring system, preferably a C₃-C₈-cycloalkyl. The cycloalkyl may be attached via any atom. Representative examples of cycloalkyl include, but are not limited to cyclopropyl, cyclobutyl, cyclopentyl, and cyclohexyl. Polycyclic cycloalkyl includes rings that can be fused, bridged, and/or spiro-fused. A cycloalkyl group can be unsubstituted or optionally substituted with one or more substituents as described herein.

"Aryl" when used alone or as part of another term means a carbocyclic aromatic group whether or not fused having the number of carbon atoms designated or if no number is designated, up to 14 carbon atoms, such as a C₆-C₁₀-aryl or C₆-C₁₄-aryl. Examples of aryl groups include phenyl, naphthyl, biphenyl, phenanthrenyl, naphthacenyl, and the like (see e.g. Lang's Handbook of Chemistry (Dean, J. A., ed) 13thed. Table 7-2 [1985]). "Aryl" also contemplates an aryl ring that is part of a fused polycyclic system, such as aryl fused to cycloalkyl as defined herein. An exemplary aryl is phenyl. An aryl group can be unsubstituted or optionally substituted with one or more substituents as described herein.

The term "heteroatom" refers to N, O, and S. Compounds of the present disclosure that contain N or S atoms can be optionally oxidized to the corresponding N-oxide, sulfoxide, or sulfone compounds. "Heteroaryl," alone or in combination with any other moiety described herein, is a monocyclic aromatic ring structure containing 5 to 10, such as 5 or 6 ring atoms, or a bicyclic aromatic group having 8 to 10 atoms, containing one or more, such as 1-4, 1-3, or 1-2, heteroatoms independently selected from the group consisting of O, S, and N. Heteroaryl is also intended to include oxidized S or N, such as sulfinyl, sulfonyl and N-oxide of a tertiary ring nitrogen. A carbon or heteroatom is the point of attachment of the heteroaryl ring structure such that a stable compound is produced. Examples of heteroaryl groups include, but are not limited to, pyridinyl, pyridazinyl, pyrazinyl, quinaoxalyl, indolizinyl, benzo[b]thienyl, quinazolinyl, purinyl, indolyl, quinolinyl, pyrimidinyl, pyrrolyl, pyrazolyl, oxazolyl, thiazolyl, thienyl, isoxazolyl, oxathiadiazolyl, isothiazolyl, tetrazolyl, imidazolyl, triazolyl, furanyl, benzofuryl, and indolyl. A preferred heteroaryl is pyridine. A heteroaryl group can be unsubstituted or optionally substituted with one or more substituents as described herein. "Heterocycloalkyl" is a saturated or partially unsaturated non-aromatic monocyclic, bicyclic, tricyclic or polycyclic ring system that has from 3 to 14, preferably 3 to 6, atoms in which 1 to 3 carbon atoms in the ring are replaced by heteroatoms of O, S or N. Polycyclic heterocycloalkyl includes rings that can be fused, bridged, and/or spiro-fused. In addition, a heterocycloalkyl is optionally fused with aryl or heteroaryl of 5-6 ring members, and includes oxidized S or N, such as sulfinyl, sulfonyl and N-oxide of a tertiary ring nitrogen. The point of attachment of the heterocycloalkyl ring is at a carbon or heteroatom such that a stable ring is retained. Examples of heterocycloalkyl groups include without limitation morpholino, tetrahydrofuranyl, dihydropyridinyl, piperidinyl, pyrrolidinyl, piperazinyl, dihydrobenzofuryl, and dihydroindolyl. A heterocycloalkyl group can be unsubstituted or optionally substituted with one or more substituents as described herein.

As used herein, alkyl-aryl or alkyl-heteroaryl indicates an alkyl group substituted with an aryl or heteroaryl, as above defined. Preferably, the alkyl chain is a C₁₋₄-alkyl, thus the substituent is referred as C₁₋₄-alkyl-aryl and C₁₋₄-alkyl-heteroaryl; more preferably said C₁₋₄-alkyl-aryl is CH₂-aryl, CH₂-CH₂-aryl, CH₂-CH₂-CH₂-aryl, CH(CH₃)CH₂-aryl, CH₂-CH₂-CH₂-CH₂-aryl, CH(CH₃)CH₂-CH₂-aryl, CH₂-CH(CH₃)CH₂-aryl; preferably the C₁₋₄-alkyl-heteroaryl is CH₂-heteroaryl, CH₂-CH₂-heteroaryl, CH₂-CH₂-CH₂-heteroaryl, CH(CH₃)CH₂-heteroaryl, CH₂-CH₂-CH₂-CH₂-heteroaryl, CH(CH₃)CH₂-CH₂-heteroaryl, CH₂-CH(CH₃)CH₂-heteroaryl, wherein preferably the heteroaryl is pyridine, more preferably is 2-pyridine.

The term "nitrile" or "cyano" can be used interchangeably and refers to a -CN group.

The term "oxo" refers to a =O atom bound to an atom that is part of a saturated or unsaturated moiety. Thus, the =O atom can be bound to a carbon, sulfur, or nitrogen atom that is part of a cyclic or acyclic moiety.

A "hydroxyl" or "hydroxy" refers to an -OH group.

Compounds described herein can exist in various isomeric forms, including configurational, geometric, and conformational isomers, including, for example, cis- or trans- conformations. The compounds may also exist in one or more tautomeric forms, including both single tautomers and mixtures of tautomers. The term "isomer" is intended to encompass all isomeric forms of a compound of this disclosure, including tautomeric forms of the compound. The compounds of the present disclosure may also exist in open-chain or cyclized forms. In some cases, one or more of the cyclized forms may result from the loss of water. The specific composition of the open-chain and cyclized forms may be dependent on how the compound is isolated, stored or administered. For example, the compound may exist primarily in an open- chained form under acidic conditions but cyclize under neutral conditions. All forms are included in the disclosure.

Some compounds described herein can have asymmetric centers and therefore exist in different enantiomeric and diastereomeric forms. A compound as described herein can be in the form of an optical isomer or a diastereomer. Accordingly, the disclosure encompasses compounds and their uses as described herein in the form of their optical isomers, diastereoisomers and mixtures thereof, including a racemic mixture. Optical isomers of the compounds of the disclosure can be obtained by known techniques such as asymmetric synthesis, chiral chromatography, simulated moving bed technology or via chemical separation of stereoisomers through the employment of optically active resolving agents.

Unless otherwise indicated, the term "stereoisomer" means one stereoisomer of a compound that is substantially free of other stereoisomers of that compound. Thus, a stereomerically pure compound having one chiral center will be substantially free of the opposite enantiomer of the compound. A stereomerically pure compound having two chiral centers will be substantially free of other diastereomers of the compound. A typical stereomerically pure compound comprises greater than about 80% by weight of one stereoisomer of the compound and less than about 20% by weight of other stereoisomers of the compound, for example greater than about 90% by weight of one stereoisomer of the compound and less than about 10% by weight of the other stereoisomers of the compound, or greater than about 95% by weight of one stereoisomer of the compound and less than about 5% by weight of the other stereoisomers of the compound, or greater than about 97% by weight of one stereoisomer of the compound and less than about 3% by weight of the other stereoisomers of the compound, or greater than about 99% by weight of one stereoisomer of the compound and less than about 1% by weight of the other stereoisomers of the compound. The stereoisomer as described above can be viewed as composition comprising two stereoisomers that are present in their respective weight percentages described herein.

If there is a discrepancy between a depicted structure and a name given to that structure, then the depicted structure controls. Additionally, if the stereochemistry of a structure or a portion of a structure is not indicated with, for example, bold or dashed lines, the structure or portion of the structure is to be interpreted as encompassing all stereoisomers of it. In some cases, however, where more than one chiral center exists, the structures and names may be represented as single enantiomers to help describe the relative stereochemistry. Those skilled in the art of organic synthesis will know if the compounds are prepared as single enantiomers from the methods used to prepare them.

As used herein, and unless otherwise specified to the contrary, the term "compound" is inclusive in that it encompasses a compound or a pharmaceutically acceptable salt, stereoisomer, and/or tautomer thereof. Thus, for instance, a compound of Formula I includes a pharmaceutically acceptable salt of a stereoisomer of the compound.

In this disclosure, a "pharmaceutically acceptable salt" is a pharmaceutically acceptable, organic or inorganic acid or base salt of a compound described herein. Representative pharmaceutically acceptable salts include, e.g., alkali metal salts, alkali earth salts, ammonium salts, water-soluble and water-insoluble salts, such as the acetate, amsonate (4,4-diaminostilbene- 2,2-disulfonate), benzenesulfonate, benzonate, bicarbonate, bisulfate, bitartrate, borate, bromide, butyrate, calcium, calcium edetate, camsylate, carbonate, chloride, citrate, clavulariate, dihydrochloride, edetate, edisylate, estolate, esylate, fiunarate, gluceptate, gluconate, glutamate, glycollylarsanilate, hexafluorophosphate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methyl sulfate, mucate, napsylate, nitrate, N-methylglucamine ammonium salt, 3-hydroxy-2-naphthoate, oleate, oxalate, palmitate, pamoate (1,1-methene-bis-2-hydroxy-3-naphthoate, einbonate), pantothenate, phosphate/diphosphate, picrate, polygalacturonate, propionate, p-toluenesulfonate, salicylate, stearate, subacetate, succinate, sulfate, sulfosaliculate, suramate, tannate, tartrate, teoclate, tosylate, triethiodide, and valerate salts. A pharmaceutically acceptable salt can have more than one charged atom in its structure. In this instance the pharmaceutically acceptable salt can have multiple counterions. Thus, a pharmaceutically acceptable salt can have one or more charged atoms and/or one or more counterions.

The present invention includes all pharmaceutically acceptable isotopically-labelled compounds of Formula (I), wherein one or more atoms are replaced by atoms having the same atomic number, but an atomic mass or mass number different from the atomic mass or mass number which predominates in nature. Examples of isotopes suitable for inclusion in the compounds of the disclosure include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S. Certain isotopically-labelled compounds of Formula (I), for example, those incorporating a radioactive isotope, are useful in drug and/or substrate tissue distribution studies. The radioactive isotopes tritium, i.e. ³H, and carbon-14, i.e. ¹⁴C, are particularly useful for this purpose in view of their ease of incorporation and ready means of detection. Substitution with heavier isotopes such as deuterium, i.e. ²H, may afford certain therapeutic advantages resulting from greater metabolic stability, for example, increased in vivo half-life or reduced dosage requirements, and hence may be preferred in some circumstances. Substitution with positron emitting isotopes, such as ¹¹C, ¹⁸F, ¹⁵O and ¹³N, can be useful in Positron Emission Topography (PET) studies for examining substrate receptor occupancy. Isotopically-labeled compounds of Formula (I) may generally be prepared by conventional techniques known to those skilled in the art or by processes analogous to those described in the accompanying Examples using an appropriate isotopically-labeled reagents in place of the non-labeled reagent previously employed. Site specific substitution of atoms having the same atomic number but an atomic mass or mass number different from the atomic mass or mass number that predominates in nature can be regarded as a substituent of a compound of the present invention. A sample of a compound having such an isotope as a substituent has at least 50% isotope incorporation at the labelled position(s). The concentration of such isotopes, e.g., deuterium, may be defined by the isotopic enrichment factor. The term "isotopic enrichment factor" as used herein means the ratio between the isotopic abundance and the natural abundance of a specified isotope. For example, if a substituent in a compound of this invention is denoted deuterium, such compound has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

The formula drawings can represent only one of the possible tautomeric, geometric, or stereoisomeric forms. It is to be understood that the invention encompasses any tautomeric, geometric, or stereoisomeric form, and mixtures thereof, and is not to be limited merely to any one tautomeric, geometric, or stereoisomeric form utilized within the formula drawings.

The terms "treat", "treating" and "treatment" refer to the amelioration or eradication of a disease or symptoms associated with a disease. In various embodiments, the terms refer to minimizing or slowing the spread, progression, or worsening of the disease resulting from the administration of one or more prophylactic or therapeutic compounds described herein to a patient with such a disease. The terms "prevent," "preventing," and "prevention" refer to the prevention of the onset, recurrence, or spread of the disease in a patient resulting from the administration of a compound described herein. The term "effective amount" refers to an amount of a compound as described herein or other active ingredient sufficient to provide a therapeutic or prophylactic benefit in the treatment or prevention of a disease or to delay or minimize symptoms associated with a disease. Further, a therapeutically effective amount with respect to a compound as described herein means that amount of therapeutic agent alone, or in combination with other therapies, that provides a therapeutic benefit in the treatment or prevention of a disease. Used in connection with a compound as described herein, the term can encompass an amount that improves overall therapy, reduces or avoids symptoms or causes of disease, or enhances the therapeutic efficacy of or is synergistic with another therapeutic agent.

A "patient" or subject" includes an animal, such as a human, cow, horse, sheep, lamb, pig, chicken, turkey, quail, cat, dog, mouse, rat, rabbit or guinea pig. In accordance with some embodiments, the animal is a mammal such as a non-primate and a primate (e.g., monkey and human). In one embodiment, a patient is a human, such as a human infant, child, adolescent or adult. In the present disclosure, the terms "patient" and "subject" are used interchangeably.

The present invention also provides a pharmaceutical formulation or a pharmaceutical composition including a disclosed compound and a pharmaceutically acceptable excipient for use in the methods of the invention.

A formulation can be prepared in any of a variety of forms for use such as for administering an active agent to a patient, who may be in need thereof, as are known in the pharmaceutical arts. For example, the pharmaceutical compositions of the present disclosure can be formulated for administration in solid or liquid form, including those adapted for the following: (1) oral administration, for example, drenches (aqueous or non-aqueous solutions or suspensions), tablets (e.g., those targeted for buccal, sublingual, and/or systemic absorption), boluses, powders, granules, and pastes for application to the tongue; (2) parenteral administration by, for example, subcutaneous, intramuscular, intraperitoneal, intravenous or epidural injection as, for example, a sterile solution or suspension, or sustained-release formulation; (3) topical administration, for example, as a cream, ointment, or a controlled- release patch or spray applied to the skin; (4) intravaginal or intrarectal administration, for example, as a pessary, cream or foam; (5) sublingual administration; (6) ocular administration; (7) transdermal administration; or (8) nasal administration.

Amounts of a disclosed compound as described herein in a formulation may vary according to factors such as the disease state, age, sex, and weight of the individual. Dosage regimens may be adjusted to provide the optimum therapeutic response. For example, a single bolus may be administered, several divided doses may be administered overtime or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier.

The specification for the dosage unit forms are dictated by and directly dependent on (a) the unique characteristics of the compound selected and the particular therapeutic effect to be achieved, and (b) the limitations inherent in the art of compounding such an active compound for the treatment of sensitivity in individuals.

Pharmaceutical compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including in the composition an agent which delays absorption, for example, monostearate salts and gelatin.

The compounds can be administered in a time release formulation, for example in a composition which includes a slow release polymer. The compounds can be prepared with carriers that will protect the compound against rapid release, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, polylactic acid and polylactic, polyglycolic copolymers (PLG). Many methods for the preparation of such formulations are generally known to those skilled in the art.

Sterile injectable solutions can be prepared by incorporating the compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

In some embodiments, a compound can be formulated with one or more additional compounds that enhance the solubility of the compound. In certain embodiments, pharmaceutical compositions described herein can be administered in combination with one or more additional therapeutic agents to treat a disorder described herein.

Compounds of the invention can be used in the treatment of patients suffering from a viral infection, e.g., a coronaviral infection. In particular, in certain embodiments, the disclosure provides a method of treating the below medical indications comprising administering to a subject in need thereof a therapeutically effective amount of a compound of formula (I) or a pharmaceutically acceptable salt thereof.

According to the invention, the viral infection is from a virus selected from the group consisting of an RNA virus, a DNA virus, preferably wherein said viral infection is an infection by an RNA virus, preferably the RNA virus is a Coronavirus, Picornavirus, Hepevirus, Influenza virus or Rhinovirus. 11) The compound for use according to claim 10 wherein the Coronavirus is selected from the group consisting of MERSr-CoV, SARS-CoV-1, SARS-CoV-2, HCoV-OC43, HCoV-229E, HCoV- NL63, and HCoV-HKU1, preferably the Coronavirus is SARS-CoV-1 and/or SARS-CoV-2. SARS-CoV2 is the highly contagious, causative viral agent of coronavirus disease 2019 (COVID19), a global pandemic.

The compounds of the invention can be used in combination with another therapeutic. In some embodiments, the method further comprises administering an additional anti-viral therapeutic. In embodiments, the anti-viral therapeutic is selected from the group consisting of ribavirin, favipiravir, ST-193, oseltamivir, zanamivir, peramivir, danoprevir, ritonavir, and remdesivir. In some embodiments, the another therapeutic is selected from the group consisting of protease inhibitors, fusion inhibitors, M2 proton channel blockers, polymerase inhibitors, 6- endonuclease inhibitors, neuraminidase inhibitors, reverse transcriptase inhibitor, aciclovir, acyclovir, protease inhibitors, arbidol, atazanavir, atripla, boceprevir, cidofovir, combivir, darunavir, docosanol, edoxudine, entry inhibitors, entecavir, famciclovir, fomivirsen, fosamprenavir, foscamet, fosfonet, ganciclovir, ibacitabine, immunovir, idoxuridine, imiquimod, inosine, integrase inhibitor, interferons, lopinavir, loviride, moroxydine, nexavir, nucleoside analogues, penciclovir, pleconaril, podophyllotoxin, ribavirin, tipranavir, trifluridine, trizivir, tromantadine, truvada, valaciclovir, valganciclovir, vicriviroc, vidarabine, viramidine, and zodovudine. In embodiments, the additional anti-viral therapeutic is selected from the group consisting of lamivudine, an interferon alpha, a VAP anti - idiotypic antibody, enfuvirtide, amantadine, rimantadine, pleconaril, aciclovir, zidovudine, fomivirsen, a morpholino, a protease inhibitor, double-stranded RNA activated caspase oligomerizer (DRACO), rifampicin, zanamivir, oseltamivir, danoprevir, ritonavir, and remdesivir.

Contemplated patients include not only humans, but other animals such as companion animals (e.g. dogs, cats), domestic animals, and wild animals (e.g. monkeys, bats, snakes).

### MATERIALS AND METHODS

### Chemistry

### General Procedure for the Synthesis of Linear Peptide Precursors

Peptide chain elongation was performed by standard chemistry, wherein the α-amino group of each amino acid was protected with a 9H-fluoren-9-ylmethoxycarbonyl group (Fmoc) or tert-butoxy-carbonyl group (Boc). To avoid any side reactions during the chain elongation steps, reactive amino acid side chains were protected with orthogonal protecting groups, effectively masking the reactive groups until removal upon specific treatment. After completion of each coupling step, the Fmoc group of the α-amino group was removed with 20% piperidine in DMF and the resin was thoroughly washed with DCM and DMF to prepare for the coupling of the subsequent Fmoc-protected amino acid derivative or intermediates (**Ia-Ic, Id**).

The α-amine protecting group used was tert-butoxy-carbonyl (Boc); the side chain amine protecting group used was allyloxycarbonyl (Alloc); the side chain carboxylic acid protecting group used was *tert*-butyl (tBu). Fmoc-protected amino acids were typically obtained from vendors such as Sigma-Aldrich, Novabiochem, Iris Biotech.

### Intermediates Ia-Ic syntheses:

### Synthetic Scheme 1

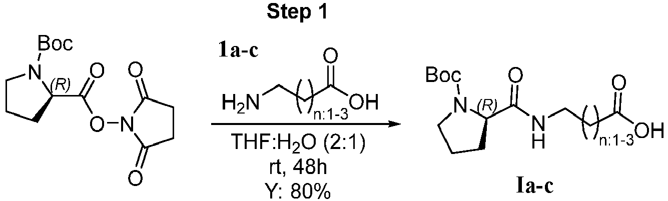

Step 1: To a mixture of Boc-D-proline *N*-hydroxysuccinimide ester (1 eq) in THF (14 mL), **1a-c** (1.2 eq) and distilled water were added at rt. The resulting mixture was stirred for 48h at rt, and then concentrated under vacuum and extracted with EtOAc. The organic layer was dried over anhydrous sodium sulfate (Na₂SO₄) and concentrated under vacuum to afford a white solid. Compounds **Ia-c** were used without further purification in the next step.

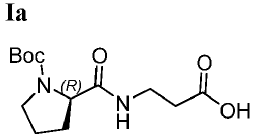

(R)-3-(1-(tert-butoxycarbonyl)pyrrolidine-2-carboxamido)propanoic acid. MS (ESI, m/z): [M-H]⁻ calcd for C₁₃H₂₁N₂O₅⁻ 285.1, found 285.2.

¹H NMR (400 MHz, DMSO) δ 12.20 (s, 1H), 7.90 (s, 1H), 4.06 - 4.01 (m, *J* = 7.1 Hz, 1H), 3.29-3.20 (m, 4H), 2.37 (t, *J* = 6.8 Hz, 2H), 1.79 - 1.66 (m, 4H), 1.33 (d, *J* = 6.8 Hz, 9H).

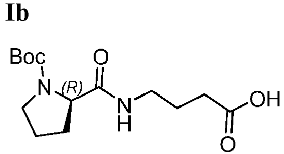

(R)-4-(1-(tert-butoxycarbonyl)pyrrolidine-2-carboxamido)butanoic acid. MS (ESI, m/z): [M-H]⁻ calcd for C₁₄H₂₃N₂O₅⁻ 299.2, found 299.3.

¹H NMR (400 MHz, CDCl₃) δ 4.26 (dd, *J* = 8.2, 3.2 Hz, 1H), 3.50 - 3.38 (m, 2H), 3.36 - 3.20 (m, 2H), 2.37 (t, *J* = 6.9 Hz, 2H), 2.29 - 1.99 (m, 2H), 1.83 (t, *J* = 5.7 Hz, 4H), 1.44 (s, 9H).

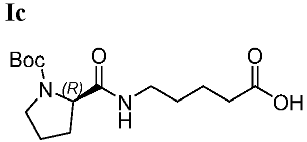

(R)-5-(1-(tert-butoxycarbonyl)pyrrolidine-2-carboxamido)pentanoic acid. MS (ESI, m/z): [M-H]⁻ calcd for C₁₅H₂₅N₂O₅⁻ 313.2, found 313.3.

¹H NMR (700 MHz, DMSO) δ 11.99 (s, 1H), 7.83 (s, 1H), 3.98 (dd, *J* = 8.3, 3.5 Hz, 1H), 3.26 (dt, *J* = 10.1, 7.0 Hz, 2H), 3.06 (dt, *J* = 12.7, 6.3 Hz, 1H), 3.03 - 2.95 (m, 1H), 2.20 (t, *J* = 7.2 Hz, 2H), 2.11 - 2.00 (m, 2H), 1.80 - 1.71 (m, 2H), 1.49 (dt, *J* = 14.6, 7.3 Hz, 4H), 1.32 (s, 9H).

### Intermediate Id Syntheses:

### Synthetic Scheme 2

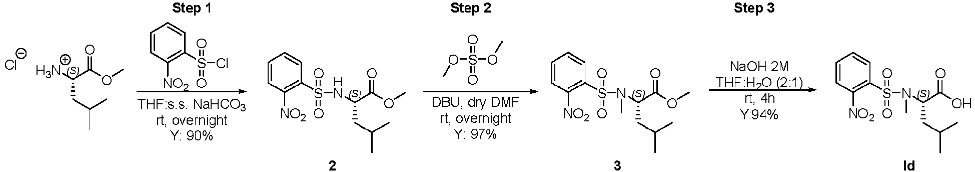

Step 1: L-Leucine methyl ester hydrochloride (1.1 eq) was suspended in a mixture of THF : s.s. NaHCO₃ (1:1), 2 -Nitrobenzenesulfonyl chloride (1 eq) was added slowly at rt. The resulting mixture was stirred for 16h at rt, and then concentrated under vacuum. The pH of the solution was adjusted to 7 with HCl 6N and extracted with EtOAc. The organic layer was dried over Na₂SO₄ and concentrated under vacuum to afford a light yellow oil. Intermediate compound (**2**) methyl ((2-nitrophenyl)sulfonyl)-L-leucinate, was used without any further purifications. MS (ESI, m/z): [M+H]⁺ calcd for C₁₃H₁₉N₂O₆S⁺ 330.1, found 285.2, 317.2, 331.3.

Step 2: To a solution of intermediate **2** (1 eq) in dry DMF, dimethyl sulfate (3 eq) and 1,5-diazabiciclo(5.4.0)undec-7-ene (DBU, 2 eq) were added. The resulting solution was left stirring for 16h at rt, and then the pH of the solution was adjusted to 7 with s.s of NaHCO₃ and extracted with EtOAc. The organic layer was dried over Na₂SO₄ and concentrated under vacuum to afford a light yellow oil. Intermediate compound Methyl N-methyl-N-((2-nitrophenyl)sulfonyl)-L-leucinate (**3**) was used without any further purifications.

Step 3: To a solution of intermediate **3** (1 eq) in THF, NaOH 2M was added at 0° C slowly. The resulting mixture was left stirring for 4h at rt, and then concentrated under vacuum. The pH of the solution was adjusted to 3 with HCl 2N and extracted with EtOAc. The organic layer was dried over Na₂SO₄ and concentrated under vacuum to afford a light yellow oil. Intermediate **Id** was used without any further purifications.

N-methyl-N-((2-nitrophenyl)sulfonyl)-L-leucine (**Id**). ¹H NMR (700 MHz, CDCl₃) δ 8.02 (dd, *J* = 7.7, 1.2 Hz, 1H), 7.72 (td, *J* = 7.6, 1.3 Hz, 1H), 7.68 (ddd, *J* = 13.4, 7.6, 1.3 Hz, 2H), 4.76 - 4.72 (m, 1H), 2.96 (s, 3H), 1.72 - 1.66 (m, 3H), 0.96 (dd, *J* = 8.3, 5.9 Hz, 6H).

### Synthetic Scheme 3

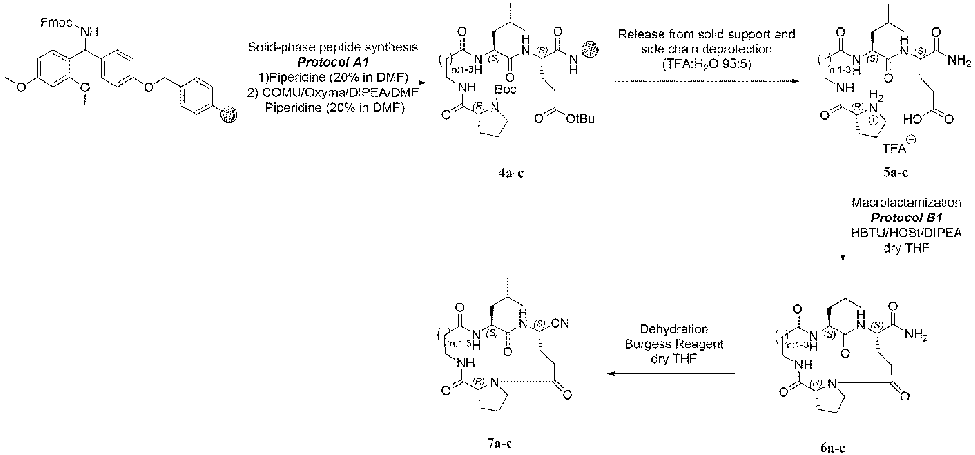

### Synthetic Scheme 4

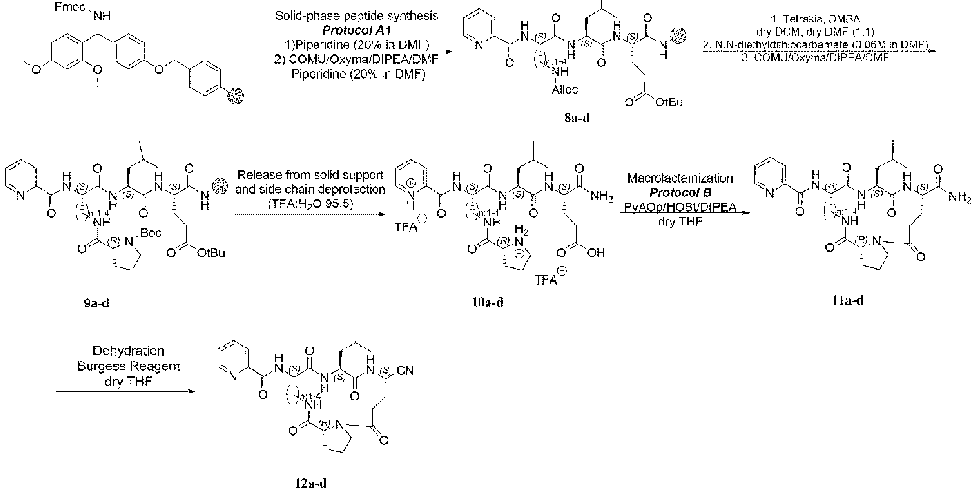

### Synthetic Scheme 5

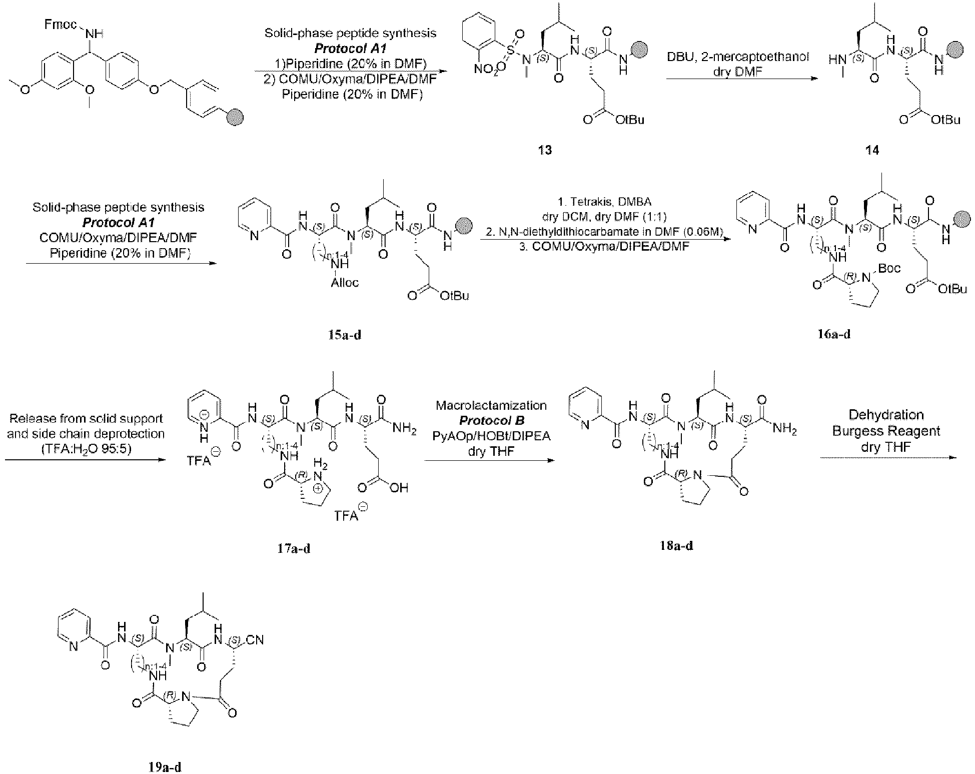

### Solid-Phase Synthesis of Peptides Protocol A1

Peptides **4a-c, 8a, 8d** and **13** were synthesized using standard solid-phase synthesis using Fmoc/tBu and FmocBoc chemistry as summarized above in Scheme **3, 4** and **5.** (1-Cyano-2-ethoxy-2-oxoethylidenaminooxy)dimethylamino-morpholino-carbenium hexafluorophosphate (COMU) with ethyl cyano(hydroxyimino)acetate (Oxyma) were used as coupling agent to form the amide bond between the free amino terminus of the resin-bound protected peptide and the carboxylic acid of the Fmoc-protected amino acid.

Fmoc-Rink-Amide resin (200-400 mesh, 0.40- 0.75 mmol/g loading, Iris Biotech) was used for synthesis. Every synthesis cycle included: Fmoc-amino acid deprotection by 20% piperidine in DMF under gentle stirring (2 × 30 min) and subsequent coupling. The amino acids or Intermediates (2 eq) were dissolved at a 0.4 mM concentration in DMF and activated with COMU (2 eq), Oxyma (2 eq) and N,N-Diisopropylethylamine (DIPEA, 4 eq), under ultrasound irradiation for 15 minutes. Reactions were typically but not exclusively performed at the 0.6 mmol scale.

The removal of Alloc protecting group from **8a-d** was performed in a mixture of dry DCM: dry DMF (1:1, 0.3 mM). Tetrakis (0.1 eq) and dimethylbarbituric acid (DMBA, 4 eq) were added and the solution was stirring for 30 min (x 2) under ultrasound irradiation. The resin was thoroughly washed with DCM and DMF and then a solution of *N,N-*diethyldithiocarbamate in DMF at the concentration of 0.06M was added and the solution was left stirring for 45 min at rt for the first cycle, and for 16 h at rt for the second cycle.

The removal of nitrobenzenesulfonamides (Nosyl) group from **13** was performed in dry DMF at 0.2 mM concentration in dry DMF. 2-mercaptoethanol (10 eq) and DBU (5 eq) were added and the solution was left stirring for 10 min (x3) under ultrasound irradiation.

### Selective Cleavage and side chain deprotection of Linear Peptides

After the complete synthesis of the linear sequences, the peptides were released from the resin and the protecting groups were removed through treatment with the cleavage cocktail of TFA/H₂O (95:5, v/v) for 3 h at rt. Then, the resin was removed by filtration and the crude peptides (**5a-c, 10a-d, 17a-d**) were precipitated using cold diethyl ether by centrifuging at 6000 rpm for 15 min × 2 to afford white solid powder (Yield: 90%).

(S)-5-amino-4-((S)-4-methyl-2-(3-((R)-pyrrolidine-2-carboxamido)propanamido)pentanamido)-5-oxopentanoic acid (**5a**). MS (ESI, m/z): [M+H]⁺ calcd for C₁₉H₃₄N₅O₆⁺ 428.2, found 428.3.

(S)-5-amino-4-((S)-4-methyl-2-(4-((R)-pyrrolidine-2-carboxamido)butanamido)pentanamido)-5-oxopentanoic acid (**5b**). MS (ESI, m/z): [M+H]⁺ calcd for C₂₀H₃₆N₅O₆⁺ 442.3, found 442.4.

(S)-5-amino-4-((S)-4-methyl-2-(5-((R)-pyrrolidine-2-carboxamido)pentanamido)pentanamido)-5-oxopentanoic acid (**5c**). MS (ESI, m/z): [M+H]⁺ calcd for C₂₁H₃₈N₅O₆⁺ 456.3, found 456.4.

(S)-5-amino-4-((S)-4-methyl-2-((S)-2-(picolinamido)-3-((R)-pyrrolidine-2 carboxamido)propanamido)pentanamido)-5-oxopentanoic acid (**10a**). MS (ESI, m/z): [M+H]⁺ calcd for C₂₅H₃₈N₇O₇⁺ 548.3, found 548.4.

(S)-5-amino-4-((S)-4-methyl-2-((S)-2-(picolinamido)-4-((R)-pyrrolidine-2-carboxamido)butanamido)pentanamido)-5-oxopentanoic acid (**10b**). MS (ESI, m/z): [M+H]⁺ calcd for C₂₆H₄₀N₇O₇⁺ 562.3, found 562.5.

(S)-5-amino-4-((S)-4-methyl-2-((S)-2-(picolinamido)-5-((R)-pyrrolidine-2-carboxamido)pentanamido)pentanamido)-5-oxopentanoic acid (**10c**). MS (ESI, m/z): [M+H]⁺ calcd for C₂₇H₄₂N₇O₇⁺ 576.3, found 576.5.

(S)-5-amino-4-((S)-4-methyl-2-((S)-2-(picolinamido)-6-((R)-pyrrolidine-2-carboxamido)hexanamido)pentanamido)-5-oxopentanoic acid (**10d**). MS (ESI, m/z): [M+H]⁺ calcd for C₂₈H₄₄N₇O₇⁺ 590.3, found 590.5.

(S)-5-amino-4-((S)-4-methyl-2-((S)-N-methyl-2-(picolinamido)-3-((R)-pyrrolidine-2-carboxamido)propanamido)pentanamido)-5-oxopentanoic acid (**17a**). MS (ESI, m/z): [M+H]⁺ calcd for C₂₆H₄₀N₇O₇⁺ 562.3, found 562.4.

(S)-5-amino-4-((S)-4-methyl-2-((S)-N-methyl-2-(picolinamido)-4-((R)-pyrrolidine-2-carboxamido)butanamido)pentanamido)-5-oxopentanoic acid (**17b**). MS (ESI, m/z): [M+H]⁺ calcd for C₂₇H₄₂N₇O₇⁺ 576.3, found 576.4.

(S)-5-amino-4-((S)-4-methyl-2-((S)-N-methyl-2-(picolinamido)-5-((R)-pyrrolidine-2-carboxamido)pentanamido)pentanamido)-5-oxopentanoic acid (**17c**). MS (ESI, m/z): [M+H]⁺ calcd for C₂₈H₄₄N₇O₇⁺ 590.3, found 590.4

(S)-5-amino-4-((S)-4-methyl-2-((S)-N-methyl-2-(picolinamido)-6-((R)-pyrrolidine-2-carboxamido)hexanamido)pentanamido)-5-oxopentanoic acid (**17d**). MS (ESI, m/z): [M+H]⁺ calcd for C₂₉H₄₆N₇O₇⁺ 604.3, found 604.4.

### Macrolactamization Protocol B1

Compounds **5a-c** were suspended at 0.04 mM concentration in dry THF and activated with 2-(1H-benzotriazol-1-yl)-1,1,3,3-tetramethyluronium hexafluorophosphate (HBTU, 1.1 eq), *N-*Hydroxybenzotriazole (HOBt, 1.1 eq) and DIPEA (3 eq) for 16h at rt, and then concentrated under vacuum. The residue was dissolved with EtOAc and washed with distilled water. The organic layers were dried over Na₂SO₄, filtered and concentrated under vacuum to afford a crude as yellow solid powder. Purification was performed by preparative reversed-phase high performance liquid chromatography (RP-HPLC) (conditions reported below). Lyophilization of combined fractions containing pure peptide resulted in the final cyclized product as a white solid powder **(6a-c,** Yield: 22%). The identity of products was confirmed by MS (ESI, m/z).

Alternatively, compounds **10a-d** and **17a-d** were suspended at 0.01 mM concentration in dry THF and activated with (7-Azabenzotriazol-1-yloxy)tripyrrolidinophosphonium hexafluorophosphate (PyAOP, 1.2 eq), HOBt (1.2 eq) and DIPEA (5 eq) for 16h at rt, and then concentrated under vacuum. The residues was purified by silica chromatography (95:5 Dichloromethane/Methanol + 1% NH₃ aq.) to afford a white solid powder (**11a-d** and **18a-d,** Yield: 60%).

### Dehydration of amide moiety

Compounds **6a-c, 11a-d** and **18a-d** were suspended at 0.03 mM concentration in dry THF. Burgess Reagent (10 eq) was added to the mixture, that was left stirring for 16h at rt. The mixture was concentrated under vacuum and the residue was purified with high performance liquid chromatography (RP-HPLC) (conditions reported below) for compounds **7a-c** while with silica chromatography (94:6 Dichloromethane/Methanol + 1% NH₃ aq.) for compounds **12a-d** and **19a-d.** Lyophilization of combined fractions containing pure peptide resulted in the final cyclized nitrile product as a white solid powder (Yield: 60%). The identity of products was confirmed by MS (ESI, m/z) see **Table 1.**

### HPLC Purification

Purification was performed by preparative reversed-phase high performance liquid chromatography (RP-HPLC) on Shimatzu LC-20AP Prep using Sunfire C18 OBD 5 µm Prep column (100 A, column size 19 * 100 mm), with mobile phase: (A) 0.1% HCOOH in HPLC water and (B) 0.1% HCOOH in HPLC acetonitrile; flow rate: 20 mL/min; UV wavelength λ = 200 nm; gradient: 5-95% B over 20 min.

Properties of all compounds are reported in Table 1 below.

| **ID NO.** | **Name** | **Exact Mass (Da)** | **Molecular Formula** | **Molecular Weight (g/mol)** | **Measured Mass (Da)** | **Observed Ion** |
|---|---|---|---|---|---|---|
| **6a** | (7S,10S,17aR)-7-isobutyl-1,5,8,13-tetraoxohexadecahydro-1H-pyrrolo[1,2-a][1,4,8,11]tetraazacyclopentadec ine-10-carboxamide | 409.23 | C₁₉H₃₁N₅O₅ | 409.48 | 410.4 | [M+H]⁺ |
| | | | | | 432.4 | [M+Na]⁺ |
| **6b** | (8S,11S,18aR)-8-isobutyl-1,6,9,14-tetraoxooctadecahydropyrrolo[1,2 -a][1,4,9,12]tetraazacyclohexadeci ne-11-carboxamide | 423.24 | C₂₀H₃₃N₅O₅ | 423.51 | 424.5 | [M+H]⁺ |
| | | | | | 446.4 | [M+Na]⁺ |
| **6c** | (9S,12S,19aR)-9-isobutyl-1,7,10,15-tetraoxooctadecahydro-1H-pyrrolo[2,1-c][1,4,9,12]tetraazacycloheptadec ine-12-carboxamide | 437.26 | C₂₁H₃₅N₅O₅ | 437.54 | 438.4 | [M+H]⁺ |
| **7a** | (7S,10S,17aR)-7-isobutyl-1,5,8,13-tetraoxohexadecahydro-1H-pyrrolo[1,2-a][1,4,8,11]tetraazacyclopentadec ine-10-carbonitrile | 391.22 | C₁₉H₂₉N₅O₄ | 391.47 | 392.4 | [M+H]⁺ |
| | | | | | 783.3 | [M+2H]/2⁺ |
| **7b** | (8S,11S,18aR)-8-isobutyl-1,6,9,14-tetraoxooctadecahydropyrrolo[1,2 -a][1,4,9,12]tetraazacyclohexadeci ne-11-carbonitrile | 405.23 | C₂₀H₃₁N₅O₄ | 405.50 | 406.3 | [M+H]⁺ |
| **7c** | (9S,12S,19aR)-9-isobutyl-1,7,10,15-tetraoxooctadecahydro-1H-pyrrolo[2,1-c] [1,4,9,12]tetraazacycloheptadec ine-12-carbonitrile | 419.25 | C₂₁H₃₃N₅O₄ | 419.52 | 420.4 | [M+H]⁺ |
| | | | | | 442.4 | [M+Na]⁺ |
| **11a** | (4S,7S,10S,17aR)-7-isobutyl-1,5,8,13-tetraoxo-4-(picolinamido)hexadecahydro-1H-pyrrolo[1,2-a][1,4,8,11]tetraazacyclopentadec ine-10-carboxamide | 529.26 | C₂₅H₃₅N₇O₆ | 529.59 | 530.5 | [M+H]⁺ |
| **11b** | (SS,8S,11S,18aR)-8-isobutyl-1,6,9,14-tetraoxo-5-(picolinamido)octadecahydropyrr olo[1,2-a][1,4,9,12]tetraazacyclohexadeci ne-11-carboxamide | 543.28 | C₂₆H₃₇N₇O₆ | 543.62 | 544.4 | [M+H]⁺ |
| **11c** | (6S,9S,12S,19aR)-9-isobutyl-1,7,10,15-tetraoxo-6-(picolinamido)octadecahydro- 1H-pyrrolo[2,1-c][1,4,9,12]tetraazacycloheptadec ine-12-carboxamide | 557.29 | C₂₇H₃₉N₇O₆ | 557.65 | 558.4 | [M+H]⁺ |
| **11d** | (7S,10S,13S,20aR)-10-isobutyl-1,8,11,16-tetraoxo-7-(picolinamido)icosahydropyrrolo[ 2,1-c][1,4,9,12]tetraazacyclooctadeci ne-13-carboxamide | 571.31 | C₂₈H₄₁N₇O₆ | 571.67 | 572.4 | [M+H]⁺ |
| **12a** | N-((4S,7S,10S,17aR)-10-cyano-7-isobutyl-1,5,8,13-tetraoxohexadecahydro-1H-pyrrolo[1,2-a][1,4,8,11]tetraazacyclopentadec in-4-yl)picolinamide | 511.25 | C₂₅H₃₃N₇O₅ | 511.58 | 512.5 | [M+H]⁺ |
| **12b** | N-((5S,8S,11S,18aR)-11-cyano-8-isobutyl-1,6,9,14-tetraoxooctadecahydropyrrolo [1,2 -a][1,4,9,12]tetraazacyclohexadeci n-5-yl)picolinamide | 525.27 | C₂₆H₃₅N₇O₅ | 525.61 | 526.5 | [M+H]⁺ |
| | | | | | 548.5 | [M+Na]⁺ |
| | | | | | 564.5 | [M+K]⁺ |
| **12c** | N-((6S,9S,12S,19aR)-12-cyano-9-isobutyl-1,7,10,15-tetraoxooctadecahydro-1H-pyrrolo[2,1-c][1,4,9,12]tetraazacycloheptadec in-6-yl)picolinamide | 539.28 | C₂₇H₃₇N₇O₅ | 539.63 | 540.5 | [M+H]⁺ |
| **12d** | N-((7S,10S,13S,20aR)-13-cyano-10-isobutyl-1,8,11,16-tetraoxoicosahydropyrrolo[2,1-c][1,4,9,12]tetraazacyclooctadeci n-7-yl)picolinamide | 553.30 | C₂₈H₃₉N₇O₅ | 553.66 | 554.4 | [M+H]⁺* |
| **18a** | (4S,7S,10S,17aR)-7-isobutyl-6-methyl-1,5,8,13-tetraoxo-4-(picolinamido)hexadecahydro-1H-pyrrolo[1,2-a][1,4,8,11]tetraazacyclopentadec ine-10-carboxamide | 543.28 | C₂₆H₃₇N₇O₆ | 543.62 | 544.4 | [M+H]⁺ |
| **18b** | (SS,8S,11S,18aR)-8-isobutyl-7-methyl-1,6,9,14-tetraoxo-5-(picolinamido)octadecahydropyrr olo[1,2-a][1,4,9,12]tetraazacyclohexadeci ne-11-carboxamide | 557.29 | C₂₇H₃₉N₇O₆ | 557.65 | 558.5 | [M+H]⁺ |
| **18c** | (6S,9S,12S,19aR)-9-isobutyl-8-methyl-1,7,10,15-tetraoxo-6-(picolinamido)octadecahydro-1H-pyrrolo[2,1-c][1,4,9,12]tetraazacycloheptadec ine-12-carboxamide | 571.31 | C₂₈H₄₁N₇O₆ | 571.67 | 572.4 | [M+H]⁺ |
| **18d** | (7S,10S,13S,20aR)-10-isobutyl-9-methyl-1,8,11,16-tetraoxo-7-(picolinamido)icosahydropyrrolo[ 2,1-c][1,4,9,12]tetraazacyclooctadeci ne-13-carboxamide | 585.32 | C₂₉H₄₃N₇O₆ | 585.70 | 586.4 | [M+H]⁺ |
| **19a** | N-((4S,7S,10S,17aR)-10-cyano-7-isobutyl-6-methyl-1,5,8,13-tetraoxohexadecahydro-1H-pyrrolo[1,2-a][1,4,8,11]tetraazacyclopentadec in-4-yl)picolinamide | 525.27 | C₂₆H₃₅N₇O₅ | 525.61 | 526.4 | [M+H]⁺ |
| **19b** | N-((5S,8S,11S,18aR)-11-cyano-8-isobutyl-7-methyl-1,6,9,14-tetraoxooctadecahydropyrrolo [1,2 -a][1,4,9,12]tetraazacyclohexadeci n-5-yl)picolinamide | 539.28 | C₂₇H₃₇N₇O₅ | 539.63 | 538.4 | [M-H]⁻ |
| | | | | | 584.0 | [M+HCOO]⁻ |
| **19c** | N-((6S,9S,12S,19aR)-12-cyano-9-isobutyl-8-methyl-1,7,10,15-tetraoxooctadecahydro-1H-pyrrolo[2,1-c][1,4,9,12]tetraazacycloheptadec in-6-yl)picolinamide | 553.30 | C₂₈H₃₉N₇O₅ | 553.66 | 554.4 | [M+H]⁺ |
| | | | | | 576.4 | [M+Na]⁺ |
| | | | | | 592.4 | [M+K]⁺ |
| **19d** | N-((7S,10S,13S,20aR)-13-cyano-10-isobutyl-9-methyl-1,8,11,16-tetraoxoicosahydropyrrolo[2,1-c][1,4,9,12]tetraazacyclooctadeci n-7-yl)picolinamide | 567.31 | C₂₉H₄₁N₇O₅ | 567.69 | 568.4 | [M+H]⁺ |

### BIOLOGY

The detection of enzymatic activity of the SARS-CoV-2 M^{pro} was performed under conditions similar to those described in ACS Pharmacol Transl Sci. 2021 Mar 11;4(3):1096-1110. doi: 10.1021/acsptsci.0c00216. eCollection 2021 Jun 11, and also reported by Zhang et al. (Zhang L.; Lin D.; Sun X.; Curth U.; Drosten C.; Sauerhering L.; Becker S.; Rox K.; Hilgenfeld R. (2020) Crystal structure of SARS-CoV-2 main protease provides a basis for design of improved α-ketoamide inhibitors. Science 368 (6489), 409-412. 10.1126/science.abb3405).

The enzymatic activity was measured in black 384 well plates (PerkinElmer), in 20 µl reaction volume containing 20 mM Tris-HCl pH 7.3, 100 mM NaCl, 1 mM EDTA, 5 mM TCEP, 0.1% BSA, 10% DMSO or inhibitor and 200 nM of purified nsp5. The reaction mixture containing the enzyme was pre-incubated for 30 min with the inhibitor at 37°C. The reaction was started adding 12 µM of substrate peptide (DABCYL-KTSAVLQSGFRKM-EDANS) and incubated for 15 min at RT. Products were measured with Victor Nivo (Perkin) at 320/480 (em/ex) nm. Experiments were performed in triplicate, the results report average and standard deviation of two independent replicates. Compound GC376 was used as positive internal control.

### SARS-CoV-2 replication assay

The African green monkey kidney cell line was previously engineered to constitutively express GFP (Vero E6-GFP) and were kindly provided by Janssen Pharmaceutical. Cells were maintained in Dulbecco's modified Eagle's medium (DMEM; Gibco) supplemented with 10% v/v fetal beef serum (FBS; Gibco), 0.075% Sodium Bicarbonate (7.5% solution, Gibco) and 1× Pen-strep (Euroclone) and kept under 5% CO2 on 37 °C. SARS-CoV-2 strain BetaCov/Belgium/GHB-03021/2020 was provided by KU Leuven. All virus-related work was carried out in certified, high-containment biosafety level-3 facilities at the University of Cagliari. Cells were seeded at 10000 cells/well in 96-well black cell-treated plates. The following day, cells were incubated with the control compounds at different concentrations and the virus at MOI 0.01. GC376 compound was used as positive control, in presence of 2 µM P-gp inhibitor CP-100356. 72 h post infection the media was removed and total well GFP fluorescence was measured with a Victor 3 with 485/535 nm excitation wavelength. The inhibition of viral replication was calculated as percentage of viral induced cytopathic effect on infected untreated controls, minus blanks (empty wells). EC50 value was calculated with Prism 9. Version 9.1.2 via non-linear regression. The experiments represent average and standard deviation of at least two independent experiments in triplicate.

For quantification of viral copy number, by RT-PCR human epithelial lung adenocarcinoma cells Calu-3 cells were plated in 96 well plates (20000 cells/well). The next day, drugs were added to cells and cells were infected with SARS-CoV-2 (MOI = 0.3) for 1 h and subsequently, the virus inoculum was removed. The cells were placed into fresh media with the indicated drugs. At 48 h post-infection, viral RNA was extracted from the supernatant with the QIAamp Viral RNA Mini Kit (Qiagen) following manufacturer instructions.

One-step RT-qPCR was performed in 20 mL to detect SARS-CoV-2 S-gene copy number using the primers: forward _GTGTTTATTTTGCTTCCACTG (SEQ ID NO 2); reverse_GGCTGAGAGACATATTCAAAA (SEQ ID NO 3) with Luna Universal One-Step RT-qPCR Kit (New England Biolabs) according to manufacturer's instructions in a CFX-96 RT-PCR (Biorad). Results report the mean and standard deviation of two independent replicates.

### MERS replication assay

Middle East respiratory syndrome (MERS) Coronavirus, strain IP/COV/MERS/Hu/France/FRA2 (Ref-SKU: 014V-02970) was provided by EVA-GLOBAL Jessica VANHOMWEGEN laboratory, Pasteur Institute. All virus-related work was carried out in certified, high-containment biosafety level-3 facilities at the University of Cagliari. Cells were seeded at 10000 cells/well in 96-well black cell-treated plates. The following day, cells were incubated with the control compounds at different concentrations and the virus at MOI 0.005. GC376 compound (Kuzikov et al., 2021) was used as positive control, in presence of 2 mM Pgp inhibitor CP-100356 (Hu et al., 2021). 4 days post infection the media was removed and total well GFP fluorescence was measured with a Victor 3 with 485/535 nm excitation wavelength. The inhibition of viral replication was calculated as percentage of viral induced cytopathic effect on infected untreated controls, minus blanks (empty wells). EC50 value was calculated with Prism 9. Version 9.1.2 via non-linear regression. The experiments represent average and standard deviation of at least two independent experiments in triplicate.

HCoV229E replication assay
hCoV229E (ATCC^{®} VR-740^{™}) was in propagated in MRC-5 cells (ATCC:CCL-171^{™}) maintained in MEM (Gibco) supplemented with 10% v/v fetal beef serum (FBS HI; Gibco), 1 mM Na Pyruvate (Euroclone), 1 mM Non Essential Amino Acids (Euroclone) and 1× Pen-strep (Euroclone) and kept under 5% CO2 on 37 °C. BEAS-2B cells were kindly provided by Pierre-Olivier Vidalain were maintained in DMEM/F-12 (Gibco), 5% FBS HI (Gibco), 1% Kanamycin (Thermo-Fisher Scientifics), at 37 °C with 5% CO2. 2*104 cells per well were seeded in transparent 96 well plate and incubated over night in order to reach 90% confluency. 24 h later cells were infected with hCoV-229E m. o.i. 0.06 in DMEM/F-12 in presence of compound or 0,1% DMSO (untreated controls). The cells were incubated for 2 h at 35 °C with 5% CO2, then the virus was removed, replaced with complete medium with or without compound, and incubated at 35 °C with 5% CO2. After 72 h, 20 µL of 3-(4,5-dimethylthiazol-2-yl)-2,5-diphenyl-2H-tetrazolium bromide (Sigma-Aldrich) dissolved in PBS at 7,5 mg/mL were added to each well and incubated at 37 °C with 5% CO2 for 1 h. Then the supernatant was removed and cells were lysed with 100 µL/well of: 10% 2-Propanol, 0,004% Triton-X-100 (Sigma-Aldrich), 0,0004% HCl, then the absorbance was read at 570 nm with a plate reader Victor Nivo5 PerkinElmer.

### Evaluation of cytotoxicity

Vero E6-GFP cells were seeded at 10,000 cells/well in 96-well black cell-treated plates, the following day, cells were incubated with the compounds and virus MOI of 0.002, 72 h post-infection the media was removed and total well GFP fluorescence was measured with a Victor 3 with 485/535 nm excitation wavelength. Cytotoxicity was calculated as a percentage of fluorescence of untreated controls, minus blanks (empty wells). CC50 value was calculated with Prism 9. Version 9.1.2 (225) via non-linear regression. The experiments represent average and standard deviation of at least two independent experiments in triplicate.

All the synthesized macrocyclic nitriles **7a-c, 12a-d, 19a-d** were tested in the FRET biochemical assay in order to evaluate their inhibitory activity against the isolated SARS-CoV- 2 M^{pro} processing a fluorescent substrate. Moreover, also macrocyclic amides **6a-c, 11a-d, 18a-d** were tested in order to indirectly assess the key role of the electrophilic warhead as a requirement for the inhibitory activity. All results are reported in table 2 below.

**Table 2: activity of the tested compounds (A: ICso less or equal to 2 µM; B: ICso comprised between > 2 µM and less or equal to 20 µM; C: higher than 20 µM)**

| **ID NO.** | **Name** | **IC₅₀ (µM)** | |
|---|---|---|---|
| | | **SARS-CoV-2** | **MERS-CoV** |
| **6a** | (7S,10S,17aR)-7-isobutyl-1,5,8,13-tetraoxohexadecahydro-1H-pyrrolo[1,2-a][1,4,8,11]tetraazacyclopentadecine-10-carboxamide | C | C |
| **6b** | (8S,11S,18aR)-8-isobutyl-1,6,9,14-tetraoxooctadecahydropyrrolo[1,2-a][1,4,9,12]tetraazacyclohexadecine-11-carboxamide | C | C |
| **6c** | (9S,12S,19aR)-9-isobutyl-1,7,10,15-tetraoxooctadecahydro-1H-pyrrolo[2,1-c][1,4,9,12]tetraazacycloheptadecine-12-carboxamide | C | C |
| **7a** | (7S,10S,17aR)-7-isobutyl-1,5,8,13-tetraoxohexadecahydro-1H-pyrrolo[1,2-a][1,4,8,11]tetraazacyclopentadecine-10-carbonitrile | C | C |
| **7b** | (8S,11S,18aR)-8-isobutyl-1,6,9,14-tetraoxooctadecahydropyrrolo[1,2-a][1,4,9,12]tetraazacyclohexadecine-11-carbonitrile | C | C |
| **7c** | (9S,12S,19aR)-9-isobutyl-1,7,10,15-tetraoxooctadecahydro-1H-pyrrolo[2,1-c][1,4,9,12]tetraazacycloheptadecine-12-carbonitrile | / | / |
| **11a** | (4S,7S,10S,17aR)-7-isobutyl-1,5,8,13-tetraoxo-4-(picolinamido)hexadecahydro-1H-pyrrolo[1,2-a][1,4,8,11]tetraazacyclopentadecine-10-carboxamide | C | C |
| **11b** | (5S,8S,11S,18aR)-8-isobutyl-1,6,9,14-tetraoxo-5-(picolinamido)octadecahydropyrrolo[1,2-a][1,4,9,12]tetraazacyclohexadecine-11-carboxamide | C | C |
| **11c** | (6 S, 9 S,12 S,19aR)-9-i sobutyl-1,7,10,15-tetraoxo-6-(picolinamido)octadecahydro-1H-pyrrolo[2,1-c][1,4,9,12]tetraazacycloheptadecine-12-carboxamide | C | C |
| **11d** | (7S,10S,13S,20aR)-10-isobutyl-1,8,11,16-tetraoxo-7-(picolinamido)icosahydropyrrolo[2,1-c][1,4,9,12]tetraazacyclooctadecine-13-carboxamide | A | C |
| **12a** | N-((4S,7S,10S,17aR)-10-cyano-7-isobutyl-1,5,8,13-tetraoxohexadecahydro-1H-pyrrolo[1,2-a][1,4,8,11]tetraazacyclopentadecin-4-yl)picolinamide | C | C |
| **12b** | N-((5S,8S,11S,18aR)-11-cyano-8-isobutyl-1,6,9,14-tetraoxooctadecahydropyrrolo[1,2-a][1,4,9,12]tetraazacyclohexadecin-5-yl)picolinamide | C | C |
| **12c** | N-((6S,9S,12S,19aR)-12-cyano-9-isobutyl-1,7,10,15-tetraoxooctadecahydro-1H-pyrrolo[2,1-c][1,4,9,12]tetraazacycloheptadecin-6-yl)picolinamide | C | C |
| **12d** | N-((7S,10S,13S,20aR)-13-cyano-10-isobutyl-1,8,11,16-tetraoxoicosahydropyrrolo[2,1-c][1,4,9,12]tetraazacyclooctadecin-7-yl)picolinamide | A | C |
| **18a** | (4S,7S,10S,17aR)-7-isobutyl-6-methyl-1,5,8,13-tetraoxo-4-(picolinamido)hexadecahydro-1H-pyrrolo[1,2-a][1,4,8,11]tetraazacyclopentadecine-10-carboxamide | C | C |
| **18b** | (5S,8S,11S,18aR)-8-isobutyl-7-methyl-1,6,9,14-tetraoxo-5-(picolinamido)octadecahydropyrrolo[1,2-a][1,4,9,12]tetraazacyclohexadecine-11-carboxamide | B | B |
| **18c** | (6S,9S,12S,19aR)-9-isobutyl-8-methyl-1,7,10,15-tetraoxo-6-(picolinamido)octadecahydro-1H-pyrrolo[2,1-c][1,4,9,12]tetraazacycloheptadecine-12-carboxamide | B | C |
| **18d** | (7S,10S,13S,20aR)-10-isobutyl-9-methyl-1,8,11,16-tetraoxo-7-(picolinamido)icosahydropyrrolo[2,1-c][1,4,9,12]tetraazacyclooctadecine-13-carboxamide | C | C |
| **19a** | N-((4S,7S,10S,17aR)-10-cyano-7-isobutyl-6-methyl-1,5,8,13-tetraoxohexadecahydro-1H-pyrrolo[1,2-a][1,4,8,11]tetraazacyclopentadecin-4-yl)picolinamide | B | C |
| **19b** | N-((5S,8S,11S,18aR)-11-cyano-8-isobutyl-7-methyl-1,6,9,14-tetraoxooctadecahydropyrrolo[1,2-a][1,4,9,12]tetraazacyclohexadecin-5-yl)picolinamide | B | C |
| **19c** | N-((6S,9S,12S,19aR)-12-cyano-9-isobutyl-8-methyl-1,7,10,15-tetraoxooctadecahydro-1H-pyrrolo[2,1-c][1,4,9,12]tetraazacycloheptadecin-6-yl)picolinamide | B | B |
| **19d** | N-((7S,10S,13S,20aR)-13-cyano-10-isobutyl-9-methyl-1,8,11,16-tetraoxoicosahydropyrrolo[2,1-c][1,4,9,12]tetraazacyclooctadecin-7-yl)picolinamide | C | C |

### REFERENCES

1. https://covid19.who.int/
2. Cannalire R. et al. 2022. Targeting SARS-CoV-2 proteases and polymerase for COVID-19 treatment: state of the art and future opportunities. J. Med. Chem. 65, 4, 2716-2746.
3. Owen D.R. et al. 2021. An oral SARS-CoV-2 Mpro inhibitor clinical candidate for the treatment of COVID-19. Science 374,1586-1593.
4. Tong, X. et al. 2023. Evaluation of in vitro antiviral activity of SARS-CoV-2 Mpro inhibitor pomotrelvir and cross-resistance to nirmatrelvir resistance substitutions. Antimicrobial Agents and Chemotherapy, 67(11); WO2021252644A1.
5. Gao, K et al. 2021. Perspectives on SARS-CoV-2 Main Protease Inhibitors. J. Med. Chem., 64(23), 16922; WO2022256434A1.
6. WO2021250648A1.
7. WO2022235605A1.
8. Yuto, U et al. 2022. Discovery of S-217622, a Noncovalent Oral SARS-CoV-2 3CL Protease Inhibitor Clinical Candidate for Treating COVID-19. J. Med. Chem., 65, 6499-6512.
9. Namoto, K et al. 2018. Structure-based design and synthesis of macrocyclic human rhinovirus 3C protease inhibitors. Bioorganic & Medicinal Chemistry Letters, 28(5), 906-909.

## Claims

1. A compound of general formula (I): 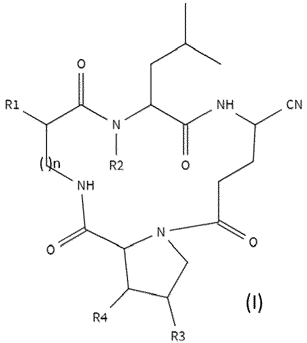 Wherein:
n is comprised between 1 and 4;
R₁ is H or RsC(=O)NH;
R₂ is H or CH₃
R₃ and R₄ are each independently selected from H, halogen, C₁₋₃alkyl, OH, C₁₋₃haloalkyl, or R₃ and
R₄ are linked together to form a fused C₃₋₆-cyclic ring or a C₃₋₆-heterocyclic ring each of said ring being optionally substituted with one or more substituents selected from methyl, halogen;
R₅ is selected from C₁₋₄alkyl, cycloalkyl, heterocycloalkyl, C₁₋₄alkyl-aryl, C₁₋₄alkyl-heteroaryl, aryl or heteroaryl wherein each of said aryl and heteroaryl is optionally substituted with one or more substituents selected from halogen, C₁₋₃alkyl, OH, C₁₋₃haloalkyl;
and stereoisomers and pharmaceutically acceptable salt thereof.

2. The compound according to claim 1, having general formula (IA): 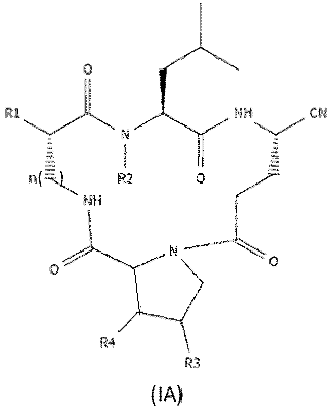 And pharmaceutically acceptable salts thereof.

3. The compound according to claims 1 or 2, having general formula (IB): 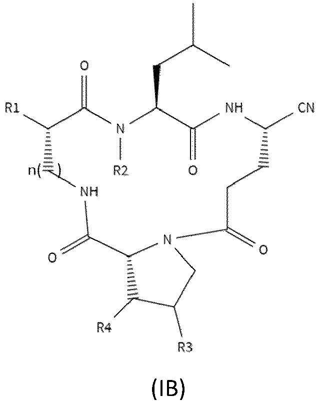 and pharmaceutically acceptable salts thereof.

4. The compound according to any one of previous claims wherein when R₁ is RsC(=O)NH.

5. The compound according to claim 4 wherein R₅ is pyridine, preferably R₅ is 2-pyridine.

6. The compound according to any one of previous claims wherein R₃ and R₄ are H.

7. The compound according to any one of previous claims selected from:
- (7S,10S,17aR)-7-isobutyl-1,5,8,13-tetraoxohexadecahydro-1H-pyrrolo[1,2-a][1,4,8,11]tetraazacyclopentadecine-10-carbonitrile
- (8S,11S,18aR)-8-isobutyl-1,6,9,14-tetraoxooctadecahydropyrrolo[1,2-a][1,4,9,12]tetraazacyclohexadecine-11-carbonitrile
- (9S,12S,19aR)-9-isobutyl-1,7,10,15-tetraoxooctadecahydro-1H-pyrrolo[2,1-c][1,4,9,12]tetraazacycloheptadecine-12-carbonitrile
- N-((4S,7S,10S,17aR)-10-cyano-7-isobutyl-1,5,8,13-tetraoxohexadecahydro-1H-pyrrolo[1,2-a][1,4,8,11]tetraazacyclopentadecin-4-yl)picolinamide
- N-((5S,8S,11S,18aR)-11-cyano-8-isobutyl-1,6,9,14-tetraoxooctadecahydropyrrolo[1,2-a][1,4,9,12]tetraazacyclohexadecin-5-yl)picolinamide
- N-((6S,9S,12S,19aR)-12-cyano-9-isobutyl-1,7,10,15-tetraoxooctadecahydro-1H-pyrrolo[2,1-c][1,4,9,12]tetraazacycloheptadecin-6-yl)picolinamide
- N-((7S,10S,13 S,20aR)-13-cyano-10-isobutyl-1,8,11,16-tetraoxoicosahydropyrrolo[2,1-c][1,4,9,12]tetraazacyclooctadecin-7-yl)picolinamide
- N-((4S,7S,10S,17aR)-10-cyano-7-isobutyl-6-methyl-1,5,8,13-tetraoxohexadecahydro-1H-pyrrolo[1,2-a][1,4,8,11]tetraazacyclopentadecin-4-yl)picolinamide
- N-((5S,8S,11S,18aR)-11-cyano-8-isobutyl-7-methyl-1,6,9,14-tetraoxooctadecahydropyrrolo[1,2-a][1,4,9,12]tetraazacyclohexadecin-5-yl)picolinamide
- N-((6S,9S,12S,19aR)-12-cyano-9-isobutyl-8-methyl-1,7,10,15-tetraoxooctadecahydro-1H-pyrrolo[2,1-c][1,4,9,12]tetraazacycloheptadecin-6-yl)picolinamide
- N-((7S,10S,13S,20aR)-13-cyano-10-isobutyl-9-methyl-1,8,11,16-tetraoxoicosahydropyrrolo[2,1-c][1,4,9,12]tetraazacyclooctadecin-7-yl)picolinamide
and pharmaceutically acceptable salts thereof.

8. The compound according to any one of previous claims being an inhibitor of the Main protease of SARS-COV2.

9. The compound according to any one of the previous claims for medical use.

10. The compound according to claim 8 for use in the treatment and/or prevention of a viral infection, preferably wherein said viral infection is an infection by an RNA virus, preferably the RNA virus is a Coronavirus, Picornavirus, Hepevirus, Influenza virus or Rhinovirus.

11. The compound for use according to claim 10 wherein the Coronavirus is selected from the group consisting of MERSr-CoV, SARS-CoV-1, SARS-CoV-2, HCoV-OC43, HCoV-229E, HCoV- NL63, and HCoV-HKU1, preferably the Coronavirus is SARS-CoV-1 and/or SARS-CoV-2

12. A pharmaceutical composition comprising the compound according to any one of claims 1 to 7 and at least one excipient and optionally a further therapeutic agent.

13. The pharmaceutical composition according to claim 12 for use in the treatment and/or prevention of a viral infection, wherein said viral infection is an infection by an RNA virus, preferably the RNA virus is a Coronavirus, Picornavirus, Hepevirus, Influenza virus, Rhinovirus.

14. The pharmaceutical composition according to claim 13 wherein the Coronavirus is selected from the group consisting of MERSr-CoV, SARS-CoV-1, SARS-CoV-2, HCoV-OC43, HCoV-229E, HCoV- NL63, and HCoV-HKU1, preferably the Coronavirus is SARS-CoV-1 and/or SARS-CoV-2

15. A method for inhibiting in vitro the M^{pro}, comprising contacting the cell with a compound according to any one of claims 1 to 7.
